# FASCICULE DE BREVET EUROPEEN

(11) **EP 1 049 445 B1**
(45) Date de publication et mention de la délivrance du brevet: **20.04.2005**
(21) Numéro de dépôt: 99936738.6
(22) Date de dépôt: 16.08.1999
(51) Int. Cl.: A61K 7/06

(54) **COMPOSITIONS CONTENANT UN POLYCONDENSAT COMPRENANT AU MOINS UN MOTIF POLYURETHANE ET/OU POLYUREE ET UNE SILICONE COMPRENANT AU MOINS UNE FONCTION CARBOXYLIQUE**
ZUSAMMENSETZUNGEN, DIE EIN POLYKONDENSAT MIT MINDESTENS EINER POLYURETHAN UND/ODER POLYHARNSTOFFEINHEIT UND EIN SILICON MIT MINDESTENS EINER CARBOXYFUNKTION ENTHALTEN.
COMPOSITIONS CONTAINING A POLYCONDENSATE COMPRISING AT LEAST A POLYURETHANE AND/OR POLYUREA UNIT AND A SILICONE COMPRISING AT LEAST A CARBOXYLIC FUNCTION

(30) Priorité: 27.08.1998 FR 9810781
(43) Date de publication de la demande: 08.11.2000
(73) Titulaire: L'OREAL, 75008 Paris (FR)
(72) Inventeur: STURLA, Jean-Michel, F-92100 Boulogne-Billancourt (FR); BREMENSON, Jean-Luc, F-75019 Paris (FR)
(74) Mandataire: Bourdeau, Françoise
(86) Numéro de dépôt international: PCT/FR1999/001993
(87) Numéro de publication internationale: WO 2000/012056

(56) Documents cités:
- EP-A- 0 838 211
- WO-A-98/20833
- DE-A- 4 241 118
- DE-A- 19 541 326
- US-A- 5 626 840

## Description

L'invention a pour objet des compositions capillaires comprenant, dans un milieu cosmétiquement acceptable, un polycondensat comprenant au moins un motif polyuréthanne et/ou polyurée et une silicone comprenant au moins une fonction carboxylique. Elle vise également un procédé pour la mise en forme ou le maintien de la coiffure comprenant la mise en oeuvre de ces compositions ainsi que leur utilisation pour la fabrication de produits capillaires, en vue d'obtenir un maintien ou une mise en forme de la coiffure.

La fixation de la coiffure est un élément important du coiffage qui consiste à maintenir la mise en forme déjà réalisée ou à mettre en forme les cheveux et à les fixer simultanément.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution peut être conditionnée par exemple dans un récipient aérosol approprié mis sous pression à l'aide d'un propulseur.

Les compositions destinées à la fixation et/ou au maintien de la coiffure présentent parfois l'inconvénient d'altérer les propriétés cosmétiques des cheveux. Ainsi, les cheveux peuvent devenir rêches et perdre leur douceur naturelle. On recherche donc des compositions de coiffage qui fixent et/ou maintiennent bien la coiffure tout en procurant de bonnes propriétés cosmétiques.

Il est connu par le brevet DE 195 41. 326 des compositions de coiffage distribuées à partir d'un dispositif aérosol qui contiennent, dans un milieu hydroalcoolique, un polymère à motif polyuréthanne en tant que polymère fixant, et un propulseur. Ces compositions, qui donnent déjà satisfaction en terme de fixation de la coiffure, peuvent toutefois être améliorées en ce qui concerne notamment les propriétés cosmétiques qu'elles confèrent aux cheveux.

De manière surprenante et inattendue, la Demanderesse a découvert, contre toute attente, qu'en associant certaines silicones à un polycondensat contenant au moins un motif polyuréthanne et/ou polyurée, il est possible de satisfaire aux exigences mentionnées ci-dessus.

L'invention a pour objet une composition capillaire comprenant, dans un milieu cosmétiquement acceptable, en proportion relative en poids par rapport au poids total de la composition, de 0,1 à 20 % d'un polycondensat comprenant au moins une séquence polyuréthanne et/ou polyurée, caractérisée par le fait qu'elle comprend en outre de 0,01 à 20 % d'au moins une silicone partiellement ou totalement neutralisée comprenant au moins une fonction carboxylique ou l'un de ses sels ou un mélange de celles-ci, le polycondensat étant formé par un arrangement de blocs, cet arrangement étant obtenu notamment à partir de:
(1) au moins un composé qui contient deux ou plus de deux atomes d'hydro gène actifs par molécule;
(2) au moins un diol ou un mélange de diols contenant des radicaux acides ou leurs sels;
(3) au moins un di- ou polyisocyanate.

Un autre objet de l'invention concerne un procédé pour la mise en forme ou le maintien de la coiffure comprenant la mise en oeuvre de cette composition.

Encore un autre objet de l'invention concerne l'utilisation de cette composition pour la fabrication de compositions capillaires, en vue d'obtenir un maintien ou une fixation de la coiffure.

Les polycondensats comprenant au moins une séquence polyuréthanne et/ou polyurée particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0619111 de la Société National Starch.

Les polycondensats utilisés conformément à l'invention peuvent être solubles dans le milieu cosmétiquement acceptable, notamment après neutralisation par une base organique ou minérale, ou encore former une dispersion dans ce milieu. La dispersion peut comprendre alors au moins 0,05 % de tensioactif permettant la mise en dispersion et le maintien en dispersion du polycondensat.

Selon l'invention, on peut utiliser tout type de tensioactif dans ladite dispersion, mais de préférence un tensioactif non ionique. La taille moyenne des particules du polycondensat dans la dispersion est de préférence comprise entre 0,1 et 1 micron.

Avantageusement, les composés (1) sont choisis dans le groupe comprenant les diols, les diamines, les polyesterols, les polyétherols ou leur mélange.

Les composés (1) qui sont préférés sont les polyéthylène et les polypropylène glycols linéaires, en particulier ceux qui sont obtenus par réaction de l'oxyde d'éthylène ou de propylène avec l'eau ou du diéthylène ou du diprapylène glycol en présence d'hydroxyde de sodium en tant que catalyseur. Ces polyglycols ont généralement un poids moléculaire compris entre environ 600 et 20000.

D'autres composés organiques préférés sont ceux qui ont des groupes mercapto, amino, carboxyle ou hydroxyle. Parmi ceux-ci, on cite plus particulièrement les composés polyhydroxy tels que les polyéther diols, les polyester diols, les polyacétal diols, les polyamide diols, les polyester polyamide diols, les poly(alkylène éther) diols, les polythioéther diols et les polycarbonate diols.

Les polyéther diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats doxyde d'alkylène. Des polyéthers appropriés sont par exemple préparés par condensation d'oxydes d'alkylène et d'alcools polyhydriques, tels que l'éthylène glycol, le 1,2-propylène glycol et le 1,4-butanediol.

Les polyester diols, polyester amides, polyamide diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylène glycol, le 1,2-propylène glycol, le 1,4-butanediol, le néopentyl glycol et l'hexane diol. On peut aussi utiliser des aminoalcools, par exemple l'éthanolamine. Des diamines appropriées pour préparer les amides polyesters sont l'éthylène diamine et l'hexaméthylène diamine.

Des polyacétals appropriés peuvent être préparés, par exemple, à partir de 1,4-butanediol ou d'hexanediol et de formaldéhyde. Des polythioéthers appropriés peuvent être préparés par exemple par réaction de condensation entre des thioglycols soit seuls ou en combinaison avec d'autres glycols tels que l'éthylène glycol, le 1,2-propylène glycol ou avec d'autres composés polyhydroxylés. Les composés polyhydroxylés contenant déjà des groupements urée ou uréthanne, des polyols naturels, qui peuvent être davantage modifiés, par exemple, l'huile de castor et les carbohydrates peuvent également être utilisés.

Plus préférentiellement, le composé du groupe (1) est un polyestérol, notamment un polyester diol formé par la réaction d'au moins un (di)-polyol (1ₐ) et d'au moins un acide (1_{b}). Le (di)- polyol (1ₐ) est en particulier choisi dans le groupe comprenant le néopentylglycol, le butanediol-1,4, l'hexanediol, l'éthylèneglycol, le diéthylène glycol, le propylèneglycol, le butylèneglycol, le néopentylglycol et (di)-polyéthytèneglycol. L'acide (1_{b}) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol propanoïque (DMPA) ou un acide carboxylique 2,2-hydroxyméthyl. En général, le composé (2) est utile en tant que bloc de couplage. En tant que composés (2), on préfère ceux comprenant au moins un poly (acide-(alpha-hydroxycarboxyliquediol)).

Les composés (2) particulièrement préférés conformément à l'invention sont ceux choisis dans le groupe comprenant le 2,2-di-(hydroxyméthyl) acide acétique, le 2,2-dihydroxyméthyl acide propionique, le 2,2-dihydroxyméthyl acide butyrique, l'acide 2,2-dihydroxyméthyl acide pentanoïque.

Le di- ou polyisocyanate (3) peut être choisi en particulier dans le groupe comprenant l'hexaméthylène diisocyanate, l'isophorondiisocyanate (IDPI), le toluylendiisocyanate, le diphénylméthane 4,4'-diisocyanate (DPMD) et le dicyclohexylméthane 4,4'-diisocyanate (DCMD), le méthylène-di-p-phényl diisocyanate, le méthylène-bis(4-cyclohexylisocyanate), l'isophorone diisocyanate, le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4- et de 2,6- toluène diisocyanate, le 2,2'-dichloro-4,4'-diisocyanato diphénylméthane, le 2,4-dibromo-1,5-diisocyanato naphtalène, le 1,4-diisocyanate butane, l'hexane-1,6-diisocyanate, le cyclohexane-1,4-diisocyanate.

Le polycondensat peut être formé à l'aide d'un composé supplémentaire (4) servant en général à allonger la chaîne du polycondensat. Ces composés (4) peuvent être choisis dans la groupe comprenant notamment les glycols saturés ou insaturés tel que l'éthylène glycol, le diéthylène glycol, le néopentylglycol, le triéthylène glycol, les aminoalcools tels que l'éthanolamine, la propanolamine, la butanolamine, les amines primaires hétérocyclique, aromatique, cycloaliphatique, et aliphatique, les diamines, les acides carboxylique tels que les acides carboxyliques aliphatique, aromatique, hétérocyclique comme l'acide oxalique, succinique, glutarique, adipique, sébacique, téréphtalique, les acides aminocarboxyliques. Les composés (4) préférés sont les diols aliphatiques.

Les polycondensats conformes à l'invention peuvent également être formés à partir de composés supplémentaires (5) ayant un squelette siliconé tels que les polysiloxanes, les polyalkylsiloxanes ou les polyarylsiloxanes notamment les polyéthylsiloxanes, les polyméthylsiloxanes et les polyphénylsiloxanes, comportant éventuellement des chaînes hydrocarbonées greffées sur les atomes de silicium.

Selon un mode de réalisation avantageux des compositions conformes à l'invention, les séquences de polyuréthanne et/ou polyurée du polymère présentent un motif répétitif de base répondant à la formule générale I ci-après:

- X - B - X - CO - NH - R - NH - CO - (I)

dans laquelle :
- X représente O et/ou NH,
- B est un radical hydrocarboné bivalent, ce radical étant substitué ou non, et
- R est un radical divalent choisi parmi les radicaux alkylène de type aromatique, aliphatique en C₁ à C₂₀, cycloaliphatique en C₁ à C₂₀, ces radicaux étant substitués ou non.

De préférence, le radical B est un radical en C₁ à C₃₀ et est porteur d'un groupement présentant une ou des fonction(s) carboxylique(s) et/ou une ou des fonctions sulfoniques, lesdites fonctions carboxyliques et/ou sulfoniques étant sous forme libre ou bien neutralisées partiellement ou totalement par une base minérale ou organique.

Le radical R est avantageusement choisi parmi les radicaux répondant aux formules suivantes: 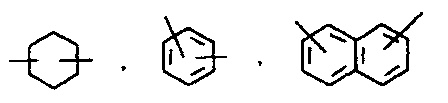 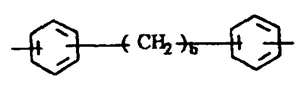 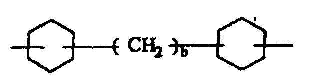 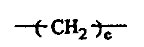 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4- et/ou 2,6-tolylène, 1,5-naphtylène, p-phénylène, méthylène- 4,4bis - cyclohéxyle et le radical divalent dérivé de l'isophorone.

Le polycondensat mis en oeuvre conformément à l'invention comprenant au moins une séquence polyuréthanne et/ou polyurée peut avantageusement comprendre en outre au moins une séquence polysiloxane dont le motif répétitif de base répond par exemple à la formule générale II ci-après:

- X - P - X - CO - NH - R - NH - CO - (II)

dans laquelle :
- P est un segment polysiloxanique,
- X représente O et/ou NH, et
- R est un radical divalent choisi parmi les radicaux alkylènes de type aromatique, aliphatique en C₁ à C₂₀, cycloaliphatique en C₁ à C₂₀, ces radicaux étant substitués ou non.

Avantageusement, le segment polysiloxanique P répond à la formule générale III ci-après: 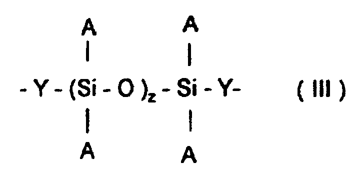 dans laquelle:
- les radicaux A, qui peuvent être identiques ou différents, sont choisis parmi d'une part les radicaux hydrocarbonés monovalents en C₁ à C₂₀ exempts ou substantiellement exempts d'insaturation éthylénique et, d'autre part, les radicaux aromatiques,
- Y représente un radical hydrocarboné bivalent, et
- z représente un nombre entier, choisi de telle sorte que le poids moléculaire moyen du segment polysiloxane soit compris entre 300 et 10 000.

En général, le radical bivalent Y est choisi parmi les radicaux alkylène de formule -(CH₂)ₐ- , dans laquelle a représente un nombre entier pouvant être compris entre 1 et 10.

Les radicaux A peuvent être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyle, en particulier le radical cyclohexyle, les radicaux aryle, notamment phényle et naphtyle, les radicaux arylalkyle, notamment benzyle et phényléthyle, ainsi que les radicaux tolyle et xylyle.

Les polycondensats utilisés conformément à l'invention peuvent être solubles dans le milieu cosmétiquement acceptable, notamment après neutralisation par une base organique ou minérale, ou encore former une dispersion dans ce milieu. Généralement, la dispersion comprend alors au moins 0,05 % de tensioactif permettant la mise en dispersion et le maintien en dispersion du polycondensat.

Selon l'invention, on peut utiliser tout type de tensioactif dans ladite dispersion, mais de préférence un tensioactif non ionique. La taille moyenne des particules du polycondensat dans la dispersion est de préférence comprise entre 0,1 et 1 micron.

La composition conforme à l'invention comprend, en proportion relative en poids par rapport au poids total de la composition, entre 0,1 et 20 % du polycondensat comprenant au moins une séquence polyuréthanne et/ou polyurée, plus avantageusement entre 1 et 15 %, et plus avantageusement encore entre 2 et 8 % de ce polycondensat.

Les silicones particulièrement visées par la présente invention sont celles décrites dans le brevet EP 0 756 860, dans la demande de brevet WO98/20833 ainsi que dans la demande de brevet française dont le numéro de dépôt est 97 16 507 et appartenant à la Demanderesse.

Au sens de la présente invention, on entend par silicone, tout polymère ou oligomère organosilicié à structure linéaire ou cyclique, ramifiée ou réticulée, de poids moléculaire variable, obtenu par polymérisation et/ou polycondensation de silanes convenablement fonctionnalisés, et constitué pour l'essentiel par une répétition de motifs principaux dans lesquels les atomes de silicium sont reliés entre eux par des atomes d'oxygène en formant une liaison siloxane ≡Si-O-Si≡, des radicaux hydrocarbonés, éventuellement substitués, étant directement liés d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle.

Selon un premier mode de réalisation des compositions conformes à l'invention, la silicone comprenant au moins une fonction carboxylique est un organopolysiloxane comprenant au moins un motif répondant à la formule I: 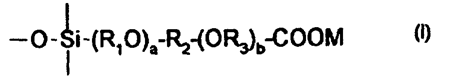 dans laquelle:
- R₁ et R₃ désignent indépendamment un radical alkylène linéaire ou ramifié ayant de 2 à 20 atomes de carbone;
- R₂ désigne un radical alkylène linéaire ou ramifié ayant de 1 à 50 atomes de carbone et comprenant éventuellement un groupement hydroxyle;
- a représente 0 ou 1;
- b est un nombre allant de 0 à 200;
- M est choisi dans le groupe comprenant l'hydrogène, les métaux alcalins
ou alcalino-terreux, NH₄, les groupements ammonium quaternaire tels que notamment les groupements mono-, di-, tri- ou tétra (alkyl C₁-C₄) ammonium.

On peut, par exemple, utiliser les organopolysiloxanes à fonction carboxylique répondant à la formule II : 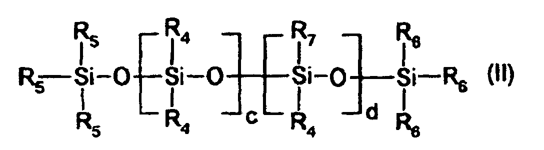 dans laquelle:
- les radicaux R₄ sont identiques ou différents et sont choisis dans le groupe comprenant les radicaux alkyles en C₁-C₂₂, linéaires ou ramifiés, les radicaux alcoxy en C₁-C₂₂ et les radicaux phénylés,
- les radicaux R₅, R₆, R₇ sont identiques ou différents et sont choisis dans le groupe comprenant les radicaux alkyles en C₁-C₂₂, linéaires ou ramifiés, les radicaux alcoxy en C₁-C₂₂, les radicaux phénylés et les radicaux (R₁O)ₐ-R₂-(OR₃)_{b}-COOM, avec la restriction que l'un au moins des radicaux R₅, R₆ ou R₇ est un radical -(R₁O)ₐ-R₂-(OR₃)_{b}-COOM,
- les radicaux R₁, R₂, R₃, a, b et M ont la même signification que dans la formule I,
- c et d sont des nombres allant de 0 à 1000, la somme c + d allant de préférence de 2 à 1000.

Parmi les silicones de formule II, on préfère les composés qui satisfont à la formule III : 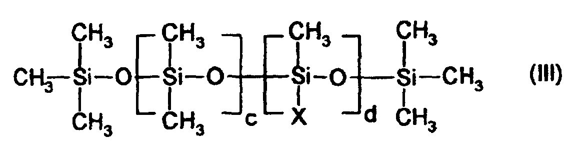 dans laquelle X est un radical -(R₁O)ₐ-R₂-(OR₃)_{b}-COOM, les radicaux R₁, R₂, R₃, a, b, d et M ayant la même signification que pour les formules I et II .

En tant que composés répondant à la formule III, on peut, par exemple, utiliser ceux commercialisés sous la marque Huile M 642, SLM 23 000/1 ou SLM 23 000/2 par la Société WACKER, ou encore sous la marque 176-12057 par la Société GENERAL ELECTRIC, ou encore sous la marque FZ 3703 par la Société OSI, ou encore sous la marque BY 16 880 par la Société TORAY SILICONE.

Selon un deuxième mode de réalisation des compositions conformes à l'invention, la silicone peut être formée par une chaîne principale de silicone répondant à la formule (≡Si-O-)ₙ sur laquelle se trouve greffé, à l'intérieur de ladite chaîne ainsi qu'éventuellement à l'une au moins de ses extrémités, au moins un groupement hydrocarboné comprenant au moins une fonction carboxylique.

La nature et/ou la quantité desdits groupements hydrocarbonés comprenant au moins une fonction carboxylique sont choisies de manière telle que le dérivé siliconé correspondant soit hydrosoluble ou hydrodispersible, après une éventuelle neutralisation des groupements à caractère anionique au moyen d'un agent alcalin.

Ces dérivés siliconés particuliers peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé anionique lui-même correctement fonctionnalisé par une fonction qui est capable de venir réagir avec le ou les groupements fonctionnels portés par ladite silicone en formant une liaison covalente; un exemple classique d'une telle réaction est la réaction d'hydrosylilation entre des groupements ≡Si-H et des groupements vinyliques CH2=CH-, ou encore la réaction entre des groupements thio-fonctionels -SH avec ces mêmes groupements vinyliques.

Des exemples de dérivés siliconés comprenant une chaîne principale de silicone sur laquelle se trouve greffé, à l'intérieur de ladite chaîne ainsi qu'éventuellement à ses extrémités, au moins un groupement hydrocarboné comprenant au moins une fonction carboxylique, qui conviennent à la mise en oeuvre de la présente invention, ainsi que leur mode particulier de préparation, sont notamment décrits dans les demandes de brevet EP 0 582152 et WO 93/23009.

Des dérivés siliconés convenant particulièrement bien à la mise en oeuvre de la présente invention sont ceux comportant dans leur structure le motif suivant: 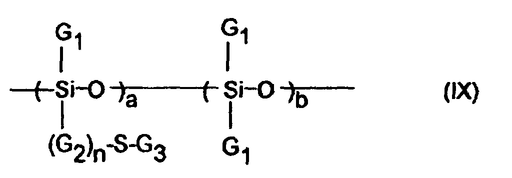 dans lequel G₁ représente l'hydrogène ou un radical alkyle en C₁-C₁₀ ou encore un radical phényle ; G₂ représente un groupe alkylène en C₁-C₁₀ ; G₃ représente un reste polymérique anionique résultant de l'(homo)polymérisation d'au moins un monomère anionique à insaturation éthylénique; n est égal à 0 ou 1; a est un nombre entier pouvant être compris entre 1 et 50; et b est un nombre entier pouvant être compris entre 10 et 350.

De préférence, le motif de formule (IX) ci-dessus présente au moins l'une, et encore plus préférentiellement l'ensemble, des caractéristiques suivantes :
- G₁ est un radical alkyle, de préférence le radical méthyle,
- n est non nul, et G₂ représente un radical divalent en C₁-C₃, de préférence un radical propylène,
- G₃ représente un radical polymérique résultant de l'(homo)polymérisation d'au moins un monomère du type acide carboxylique insaturé, de préférence l'acide acrylique et/ou méthacrylique.

Le taux de groupements carboxylate dans le polymère final est de préférence compris entre 1 mole de carboxylate pour 200 g de polymère et 1 mole de carboxylate pour 5000 g de polymère.

De préférence, la masse moléculaire en nombre du polymère siliconé est comprise entre 10 000 et 1 000 000 environ, et encore plus préférentiellement entre 10 000 et 100 000 environ.

Des exemples de dérivés siliconés convenant particulièrement bien à la réalisation de la présente invention sont notamment ceux vendus par la Société 3M sous la marque Silicone "plus" Polymer VS 80. Ces produits correspondent à des polydiméthylsiloxanes (PDMS) sur lesquels sont greffés, par l'intermédiaire d'un chainon de raccordement de type thiopropylène, des motifs polymères mixtes du type acide poly(méth)acrylique et du type ester poly(méth)acrylate de butyle. Ces produits peuvent être classiquement obtenus par copolymérisation radicalaire entre d'une part une silicone de type polydiméthylsiloxane préalablement fonctionalisée par des groupements thiopropyl et, d'autre part, un mélange de monomères constitué d'acide (méth)acrylique et de (méth)acrylate de butyle.

D'autres silicones convenant particulièrement bien pour la mise en oeuvre de la présente invention sont les silicones comprenant au moins un substituant contenant au moins deux groupements, identiques ou différents, choisis parmi les acides carboxyliques ou leurs sels, les amides et les esters, l'un au moins de ces groupements étant un acide carboxylique ou ses sels.

Ces silicones comprennent de préférence au moins un motif de formule IV:

ZRₐSiO_{(3-a)/2} (IV)

dans laquelle formule IV Z est un radical répondant à la formule V suivante: 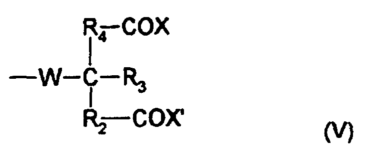 dans laquelle formule V :
- W, R₂ et R₄, identiques ou différents, sont choisis parmi une liaison covalente et un radical alkylène linéaire ou ramifié ayant de 1 à 6 atomes de carbone pouvant comprendre un groupement hydroxyle,
- R₃ désigne un atome d'hydrogène, un radical alkyle linéaire ou ramifié en C₁-C₆, X et X', identiques ou différents, sont choisis parmi les radicaux OM, NR₅R₆ et OR₇,
- M désigne un atome d'hydrogène, un métal alcalin (par exemple Na⁺, K⁺), NH₄,⁺ les groupements ammonium comportant un reste choisi dans le groupe comprenant les aminoacides basiques tels que la lysine, l'arginine, la sarcosine, l'omithine, la citrulline et les aminoalcools tels que la monoéthanolamine, la diéthanolamine, la triéthanolamine, la glucamine, la N-méthyl glucamine, l'amino-3 propanediol-1,2,
- R₅ et R₆, identiques ou différents, sont choisis dans le groupe comprenant l'hydrogène et les alkyles linéaires ou ramifiés en C₁- C₆ ou bien R₅ et R₆ peuvent former ensemble un hétérocycle à 5 ou 6 chaînons tel que la morpholine,
- R₇ est choisi parmi les radicaux alkyles linéaires ou ramifiés en C₁-C₃₀,
- l'un au moins des groupements X et X' désigne OM,

Dans la formule IV, les radicaux R, identiques ou différents, sont choisis parmi les radicaux alkyles notamment en C₁-C₁₀ et en particulier méthyle, les radicaux fluoroalkyles notamment en C₁-C₁₀, les radicaux aryles en C₆-C₁₂ et en particulier phényle; a est choisi parmi 0, 1 et 2 et de préférence 1 ou 2.

De façon préférentielle, on utilise les silicones comprenant au moins un motif de formule générale IV qui répondent à au moins une des, et de préférence à toutes les conditions suivantes :
- W désigne une liaison covalente,
- R₃ désigne un atome d'hydrogène,
- R désigne le radical méthyle,
- X et X' sont choisis parmi OM et NR₅R_{6,}
- R₂ et R₄, identiques ou différents, sont choisis parmi une liaison covalente et un radical méthyle.

Les autres unités de la silicone sont de préférence choisies parmi celles de formule VI:

R_{b}SiO_{(4-b)/2} (VI)

dans laquelle R a la même signification que pour la formule IV et b est égal à 0, 1, 2 ou 3 et de préférence égal à 2 ou 3.

Les silicones comprenant au moins une unité de formule IV sont notamment décrites dans le brevet US 4 931 062. De telles silicones sont par exemple commercialisées sous la marque SLM 23105 par la société WACKER et sous la marque DENSODRIN OF par la société BASF.

La proportion relative en poids, par rapport au poids total de la composition, en silicone ou en mélange de silicones est comprise entre 0,01 et 20 %, plus avantageusement entre 0,01 et 10 %, et plus avantageusement encore entre 0,05 et 5 %.

La composition conforme à l'invention peut se présenter sous la forme d'une lotion ou d'un gel. Elle peut s'appliquer par pulvérisation, à partir d'un flacon pompe ou d'un aérosol.

Le conditionnement sous forme aérosol est spécialement pratique pour l'utilisateur qui obtient sans difficulté une répartition assez homogène du produit. Toutefois, ce type de conditionnement présente l'inconvénient de donner lieu à un dégagement de composés organiques volatiles (VOC) nocifs pour l'environnement. Ils proviennent notamment de la quantité de solvant organique mis en oeuvre et du gaz propulseur choisi pour fabriquer la composition. On porte donc un intérêt tout particulier à la réalisation de compositions cosmétiques conditionnées sous forme aérosol pour lesquelles la quantité de composés organiques volatiles rejetés est de plus en plus faible.

La qualité de la pulvérisation obtenue au moyen d'un dispositif aérosol, c'est-à-dire essentiellement la distribution des gouttelettes dans l'espace à la sortie de la buse, dépend fortement de la constitution chimique de la composition mise en oeuvre. On porte donc un intérêt tout particulier à la formulation de compositions cosmétiques qui donnent lieu à une qualité de pulvérisation optimale.

On utilise avantageusement entre 7,5 et 70 % d'un solvant organique, plus avantageusement entre 10 et 50 %, et plus avantageusement encore entre 10 et 25 % en proportion relative en poids par rapport au poids total de la composition.

Conformément à l'invention, le solvant organique est notamment choisi dans le groupe comprenant les alcools inférieurs en C₁ à C₄ tels que l'éthanol, l'isopropanol, l'acétone, la méthyléthylcétone, l'acétate de méthyle, l'acétate de butyle, l'acétate d'éthyle, le diméthoxyéthane, le diéthoxyéthane et leurs mélanges. De manière préférentielle, on utilise l'éthanol.

Selon un mode de réalisation avantageux de la composition conforme à l'invention, elle comprend en proportion relative en poids par rapport au poids total de la composition entre 15 et 85 % d'un gaz propulseur, de préférence entre 25 et 60 %, et encore plus avantageusement entre 30 et 50 %.

Conformément à l'invention, on utilise, de préférence, comme gaz propulseur, un gaz soluble ou non dans la composition tel que le diméthyl éther, les hydrocarbures fluorés ou non, les gaz liquéfiés usuels ou un mélange de ces gaz propulseurs. Encore plus avantageusement, on utilise le diméthyléther.

De manière avantageuse, les concentrations et la nature des différents composants est choisie de façon à diminuer les teneurs en composés organiques volatiles (VOC).

Les compositions conformes à l'invention peuvent par ailleurs contenir des additifs cosmétiques conventionnels choisis notamment parmi les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères hydrocarbonés, des silicones volatiles ou non supplémentaires et autres que celles décrites précédemment, les protéines et les vitamines.

En particulier, il peut être avantageux d'ajouter à la composition d'autres polymères fixants tels que des polymères fixants non ioniques, anioniques, cationiques ou amphotères.

L'invention pourra être mieux comprise à l'aide de l'exemple non limitatif qui suit et qui constitue un mode de réalisation avantageux des compositions conformes à l'invention.

### Exemple:

On réalise la composition ci-après pour laquelle les pourcentages sont des pourcentages relatifs en poids.
- Polycondensat polyester acide lactique / éthylène glycol P(MIS-EG) - acide di- méthylol propanoïque (DMPA) - isophoronediisocyanate 4 %
- Silicone '"'Plus" Polymer VS 80 0,2 %
- Aminométhyl propanol qs neutralisation
- Ethanol 15 %
- Diméthyléther 35 %
- Eau déminéralisée qsp 100 %

## Revendications

1. Composition capillaire comprenant, dans un milieu cosmétiquement acceptable, en proportion relative en poids par rapport au poids total de la composition, de 0,1 à 20 % d'un polycondensat comprenant au moins une séquence polyuréthanne et/ou polyurée, **caractérisée par le fait qu'**elle comprend en outre de 0,01 à 20 % d'au moins une silicone partiellement ou totalement neutralisée comprenant au moins une fonction carboxylique ou l'un de ses sels ou un mélange de celles-ci, le polycondensat étant formé par un arrangement de blocs obtenu à partir de:
(1) au moins un composé qui contient deux ou plus de deux atomes d'hydro gène actifs par molécule;
(2) au moins un diol ou un mélange de diols contenant des radicaux acides ou leurs sels;
(3) au moins un di- ou polyisocyanate.

2. Composition selon la revendication 1, **caractérisée par le fait que** les composés (1) sont choisis dans le groupe comprenant les diols, les diamines, les polyesterols, les polyétherols ou leur mélange.

3. Composition selon la revendication 1, **caractérisée par le fait que** le composé (2) est un acide carboxylique 2,2-hydroxyméthyl.

4. Composition selon la revendication 1, **caractérisée par le fait que** le composé (3) est choisi dans la groupe comprenant l'hexaméthylène diisocyanate, l'isophorondiisocyanate, le toluylendiisocyanate, le diphénylméthane 4,4'-diisocyanate, le dicyclohexylméthane 4,4'-diisocyanate, le méthylène-di-p-phényl diisocyanate, le méthylène-bis(4-cyclohexylisocyanate), l'isophorone diisocyanate, le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4- et de 2,6- toluène diisocyanate, le 2,2'-dichloro-4,4'-diisocyanato diphénylméthane, le 2,4-dibromo-1,5-diisocyanato naphtalène, le 1,4-diisocyanate butane, l'hexane-1,6-diisocyanate et le cyclohexane-1,4-diisocyanate.

5. Composition selon la revendication 1, **caractérisée par le fait que** le polycondensat est formé à partir d'au moins un composé supplémentaire ayant un squelette siliconé et choisi dans le groupe comprenant les polysiloxanes, les polyalkylsiloxanes ou les polyarylsiloxanes.

6. Composition selon la revendication 5, **caractérisée par le fait que** le polycondensat est formé à partir d'au moins un composé supplémentaire ayant un squelette siliconé choisi dans le groupe comprenant les polyéthylsiloxanes, les polyméthylsiloxanes et les polyphénylsiloxanes, comportant éventuellement des chaînes hydrocarbonées greffées sur les atomes de silicium.

7. Composition selon la revendication 1, **caractérisée par le fait que** les séquences de polyuréthanne et/ou polyurée du polycondensat présentent un motif répétitif de base répondant à la formule générale l' ci-après:
- X' - B - X' - CO - NH - R - NH - CO - (I')
dans laquelle:
- X' représente O et/ou NH,
- B est un radical hydrocarboné bivalent, ce radical étant substitué ou non, et
- R est un radical divalent choisi parmi les radicaux alkylène de type aromatique, aliphatique en C₁ à C₂₀, cycloaliphatique en C₁ à C₂₀, ces radicaux étant substitués ou non.

8. Composition selon la revendication 7, **caractérisée par le fait que** B est un radical hydrocarboné bivalent en C₁ à C₃₀.

9. Composition selon la revendication 7, **caractérisée par le fait que** le radical R est choisi dans le groupe comprenant les radicaux hexaméthylène, 4,4'-biphényléneméthane, 2,4- et/ou 2,6-tolylène, 1,5-naphtylène, p-phénylène, méthylène- 4,4bis - cyclohéxyle et le radical divalent dérivé de l'isophorone.

10. Composition selon la revendication 1, **caractérisée par le fait que** le polycondensat présente un motif répétitif de base répondant à la formule (II'):
- X' - P - X' - CO - NH - R - NH - CO - (II')
dans laquelle :
- P est un segment polysiloxanique,
- X' représente O et/ou NH, et
- R est un radical divalent choisi parmi les radicaux alkylène de type aromatique, aliphatique en C₁ à C₂₀, cycloaliphatique en C₁ à C₂₀, ces radicaux étant substitués ou non.

11. Composition selon l'une quelconque des revendications précédentes, **caractérisée par le fait que** la silicone est un organopolysiloxane comprenant au moins un motif répondant à la formule I: 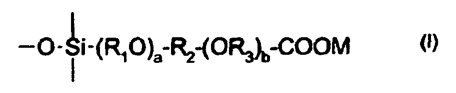 dans laquelle:
- R₁ et R₃ désignent indépendamment un radical alkylène linéaire ou ramifié ayant de 2 à 20 atomes de carbone;
- R₂ désigne un radical alkylène linéaire ou ramifié ayant de 1 à 50 atomes de carbone et comprenant éventuellement un groupement hydroxyle;
- a représente 0 ou 1;
- b est un nombre allant de 0 à 200;
- M est choisi dans le groupe comprenant l'hydrogène, les métaux alcalins ou alcalino-terreux, NH₄, les groupements ammonium quaternaire tels que notamment les groupements mono-, di-, tri- ou tétra (alkyl C₁-C₄) ammonium.

12. Composition selon l'une quelconque des revendications 1 à 10, **caractérisée par le fait que** la silicone est formée par une chaîne principale répondant à la formule (≡Si-O-)ₙ sur laquelle se trouve greffé, à l'intérieur de ladite chaîne ainsi qu'éventuellement à l'une au moins de ses extrémités, au moins un groupement hydrocarboné comprenant au moins une fonction carboxylique.

13. Composition selon la revendication 12, **caractérisée par le fait que** la silicone comprend le motif IX suivant: 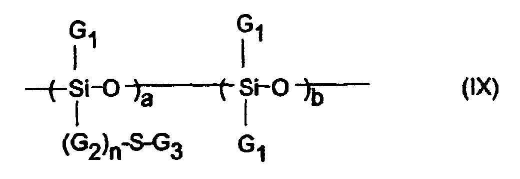 dans lequel G₁ représente l'hydrogène ou un radical alkyle en C₁-C₁₀ ou encore un radical phényle ; G₂ représente un groupe alkylène en C₁-C₁₀ ; G₃ représente un reste polymérique anionique résultant de l'(homo)polymérisation d'au moins un monomère anionique à insaturation éthylénique; n est égal à 0 ou 1 ; a est un nombre entier pouvant être compris entre 1 et 50; et b est un nombre entier pouvant être compris entre 10 et 350.

14. Composition selon rune quelconque des revendications 1 à 10, **caractérisée par le fait que** la silicone comprend au moins un motif de formule IV:
ZRₐSiO_{(3-a)/2} (IV)
dans laquelle Z est un radical répondant à la formule V suivante: 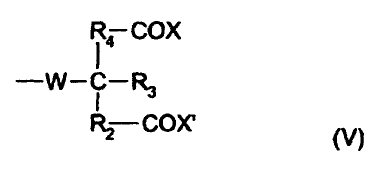 dans laquelle:
- W, R₂ et R₄, identiques ou différents, sont choisis parmi une liaison covalente et un radical alkylène linéaire ou ramifié ayant de 1 à 6 atomes de carbone pouvant comprendre un groupement hydroxyle,
- R₃ désigne un atome d'hydrogène, un radical alkyle linéaire ou ramifié en C₁-C₆, X et X', identiques ou différents, sont choisis parmi les radicaux OM, NR₅R₆ et OR₇,
- M désigne un atome d'hydrogène, un métal alcalin (par exemple Na⁺, K⁺), NH₄,⁺ les groupements ammonium comportant un reste choisi dans le groupe comprenant les aminoacides basiques tels que la lysine, l'arginine, la sarcosine, l'omithine, la citrulline et les aminoalcools tels que la monoéthanolamine, la diéthanolamine, la triéthanolamine, la glucamine, la N-méthyl glucamine, l'amino-3 propanediol-1,2,
- R₅ et R₆, identiques ou différents, sont choisis dans le groupe comprenant l'hydrogène et les alkyles linéaires ou ramifiés en C₁- C₆ ou bien R₅ et R₆ peuvent former ensemble un hétérocycle à 5 ou 6 chaînons tel que la morpholine,
- R₇ est choisi parmi les radicaux alkyles linéaires ou ramifiés en C₁ à C₃₀,
- l'un au moins des groupements X ou X' désigne OM,
les radicaux R de la formule IV, identiques ou différents, étant choisis dans le groupe comprenant les radicaux alkyles en C₁ à C₁₀, les radicaux fluoroalkyles en C₁ à C₁₀ et les radicaux aryles en C₆ à C₁₂; a étant un entier valant 0, 1 ou 2.

15. Composition selon l'une quelconque des revendications précédentes, **caractérisée par le fait que** la proportion relative en poids en silicone ou en mélange de silicones est comprise entre 0,01 et 10 %, et plus avantageusement entre 0,05 et 5 %.

16. Composition selon la revendication précédente 15, **caractérisée par le fait que** la proportion relative en poids en silicone ou en mélange de silicones est comprise entre 0,05 et 5 %.

17. Composition selon rune quelconque des revendications précédentes, **caractérisée par le fait qu'**elle comprend, en proportion relative en poids, entre 1 et 15 % du polycondensat comprenant au moins une séquence polyuréthanne et/ou polyurée, et plus avantageusement entre 2 et 8 %.

18. Composition selon l'une quelconque des revendications précédentes, **caractérisée par le fait qu'**elle comprend, en proportion relative en poids, entre 2 et 8 % du polycondensat comprenant au moins une séquence polyuréthanne et/ou polyurée.

19. Composition selon l'une quelconque des revendications précédentes, **caractérisée par le fait qu'**elle est véhiculée via un flacon pompe ou un dispositif aérosol.

20. Composition selon la revendication 19 véhiculée via un dispositif aérosol, **caractérisée par le fait qu'**elle comprend (i) un solvant organique présent à une concentration relative en poids comprise entre 10 et 50 %.

21. Composition selon la revendication 20 véhiculée via un dispositif aérosol, **caractérisée par le fait qu'**elle comprend (i) un solvant organique présent à une concentration relative en poids comprise entre 10 et 25 %.

22. Composition selon la revendication 21 véhiculée via un dispositif aérosol, **caractérisée par le fait qu'**elle comprend en outre (ii) un gaz propulseur présent à une concentration relative en poids comprise entre 15 et 85 %.

23. Composition selon la revendication 22 véhiculée via un dispositif aérosol, **caractérisée par le fait qu'**elle comprend en outre (ii) un gaz propulseur présent à une concentration relative en poids comprise entre 25 et 60 %.

24. Composition selon la revendication 23 véhiculée via un dispositif aérosol, **caractérisée par le fait qu'**elle comprend en outre (ii) un gaz propulseur présent à une concentration relative en poids comprise entre 30 et 50 %.

25. Composition selon l'une quelconque des revendications précédentes, **caractérisée par le fait qu'**elle contient en outre des additifs cosmétiques conventionnels choisis dans le groupe comprenant les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères hydrocarbonés, des silicones volatiles ou non supplémentaires, les protéines et les vitamines.

26. Composition selon l'une quelconque des revendications précédentes, **caractérisée par le fait qu'**elle contient au moins un polymère fixant additionnel choisi dans le groupe comprenant les polymères fixant non ioniques, cationiques, anioniques ou amphotères.

27. Dispositif aérosol constitué par un récipient contenant une composition aérosol selon la revendication 1 ainsi qu'un moyen de distribution de ladite composition aérosol.

28. Procédé pour la mise en forme ou le maintien de la coiffure, **caractérisé par le fait qu'**il comprend l'application d'une composition conforme à l'une quelconque des revendications 1 à 26.

29. Utilisation d'une composition selon l'une quelconque des revendications 1 à 26 pour la fabrication d'une composition capillaire, en vue d'obtenir un maintien ou une mise en forme de la coiffure.

## Patentansprüche

1. Zusammensetzung für die Haarbehandlung, die in einem kosmetisch akzeptablen Medium in einer relativen Gewichtsmenge, bezogen auf das Gesamtgewicht der Zusammensetzung, 0,1 bis 20 % Polykondensat enthält, das mindestens eine Polyurethansequenz und/oder Polyharnstoffsequenz umfasst, **dadurch gekennzeichnet, dass** sie ferner 0,01 bis 20 % mindestens eines teilweise oder vollständig neutralisierten Silicons, das mindestens eine Carboxyfunktion besitzt, oder eines seiner Salze oder eines Gemisch solcher Verbindungen enthält, wobei das Polykondensat aus einer blockförmigen Anordnung gebildet wird, die aus den folgenden Verbindungen erhalten wird:
(1) mindestens einer Verbindung, die zwei oder mehr als zwei aktive Wasserstoffatome pro Molekül enthält,
(2) mindestens einem Diol oder einem Gemisch von Diolen, die saure Gruppen oder deren Salze enthalten, und
(3) mindestens einem Di- oder Polyisocyanat.

2. Zusammensetzung nach Anspruch 1, **dadurch gekennzeichnet, dass** die Verbindungen (1) unter den Diolen, Diaminen, Polyesterolen, Polyetherolen oder deren Gemischen ausgewählt sind.

3. Zusammensetzung nach Anspruch 1, **dadurch gekennzeichnet, dass** die Verbindung (2) eine 2,2-Hydroxymethylcarbonsäure ist.

4. Zusammensetzung nach Anspruch 1, **dadurch gekennzeichnet, dass** die Verbindung (3) unter Hexamethylendiisocyanat, Isophorondiisocyanat, Toluylendiisocyanat, Diphenylmethan-4,4'-diisocyanat, Dicyclohexylmethan-4,4'-diisocyanat, Methylendi-p-phenyldiisocyanat, Methylen-bis(4-cyclohexylisocyanat), Isophorondiisocyanat, Toluoldiisocyanat, 1,5-Naphthalindiisocyanat, 4,4'-Diphenylmethandiisocyanat, 2,2'-Dimethyl-4,4'diphenylmethandiisocyanat, 1,3-Phenylendiisocyanat, 1,4-Phenylendiisocyanat, Gemischen von 2,4- und 2,6-Toluoldiisocyanat, 2,2'-Dichlor-4,4'-diisocyanato-diphenylmethan, 2,4-Dibrom-1,5-diisocyanato-naphthalin, 1,4-Diisocyanatobutan, Hexan-1,6-diisocyanat und Cyclohexan-1,4-diisocyanat ausgewählt ist.

5. Zusammensetzung nach Anspruch 1, **dadurch gekennzeichnet, dass** das Polykondensat ausgehend von mindestens einer zusätzlichen Verbindung gebildet wird, welche ein Silicongerüst aufweist und unter den Polysiloxanen, Polyalkylsiloxanen und Polyarylsiloxanen ausgewählt ist.

6. Zusammensetzung nach Anspruch 5, **dadurch gekennzeichnet, dass** das Polykondensat ausgehend von mindestens einer zusätzlichen Verbindung gebildet wird, welche ein Silicongerüst aufweist und unter den Polyethylsiloxanen, Polymethylsiloxanen und Polyphenylsiloxanen ausgewählt ist, die gegebenenfalls auf die Siliciumatome gepfropfte Kohlenwasserstoffketten enthalten.

7. Zusammensetzung nach Anspruch 1, **dadurch gekennzeichnet, dass** die Polyurethansequenzen und/oder Polyharnstoffsequenzen des Polykondensat eine wiederkehrende Grundeinheit der folgenden allgemeinen Formel (I') besitzen:
-X'-B-X'-CO-NH-R-NH-CO- (I'),
worin bedeuten:
- X' O und/oder NH,
- B eine zweiwertige Kohlenwasserstoffgruppe, wobei diese Gruppe unsubstituiert oder substituiert vorliegt, und
- R eine zweiwertige Gruppe, die unter den Alkylengruppen vom aromatischen Typ, vom aliphatischen Typ mit 1 bis 20 Kohlenstoffatomen und vom cycloaliphatischen Typ mit 1 bis 20 Kohlenstoffatomen ausgewählt ist, wobei diese Gruppen substituiert oder unsubstituiert sein können.

8. Zusammensetzung nach Anspruch 7, **dadurch gekennzeichnet, dass** die Gruppe B eine zweiwertige C₁₋₃₀-Kohlenwasserstoffgruppe ist.

9. Zusammensetzung nach Anspruch 7, **dadurch gekennzeichnet, dass** die Gruppe R unter den Gruppen Hexamethylen, 4,4'-Biphenylenmethan, 2,4- und/oder 2,6-Toluylen, 1,5-Naphthylen, p-Phenylen, Methylen-4,4-bis-cyclohexyl und der von Isophoron abgeleiteten zweiwertigen Gruppe ausgewählt ist.

10. Zusammensetzung nach Anspruch 1, **dadurch gekennzeichnet, dass** das Polykondensat eine wiederkehrende Grundeinheit der folgenden Formel (II') enthält:
-X'-P-X'-CO-NH-R-NH-CO- (II'),
worin bedeuten:
- P eine Polysiloxansegment,
- X' O und/oder NH,
- R eine zweiwertige Gruppe, die unter den Alkylengruppen vom aromatischen Typ, vom aliphatischen Typ mit 1 bis 20 Kohlenstoffatomen und vom cycloaliphatischen Typ mit 1 bis 20 Kohlenstoffatomen ausgewählt ist, wobei diese Gruppen substituiert oder unsubstituiert sein können.

11. Zusammensetzung nach einem der vorhergehenden Ansprüche, **dadurch gekennzeichnet, dass** das Silicon ein Organopolysiloxan ist, das mindestens eine Einheit (I) enthält: 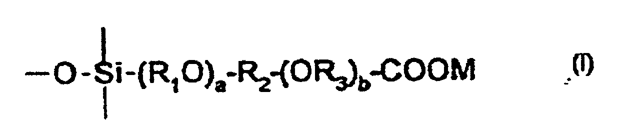 worin bedeuten:
- die Gruppen R₁ und R₃ unabhängig voneinander geradkettige oder verzweigte Alkylengruppen mit 2 bis 20 Kohlenstoffatomen,
- die Gruppe R₂ eine geradkettige oder verzweigte Alkylengruppe mit 1 bis 50 Kohlenstoffatomen, die eine Hydroxygruppe enthalten kann,
- a 0 oder 1,
- b eine Zahl im Bereich von 0 bis 200,
- M Wasserstoff, ein Alkalimetall, ein Erdalkalimetall, NH₄ oder eine quartäre Ammoniumgruppe, insbesondere Mono-, Di-, Tri- oder Tetra-C₁₋₄-alkylammoniumgruppen.

12. Zusammensetzung nach einem der Ansprüche 1 bis 10, **dadurch gekennzeichnet, dass** das Silicon aus einer Siliconhauptkette der Formel (≡Si-O-)ₙ) besteht, auf die in der Kette sowie gegebenenfalls an mindestens einem ihrer Enden mindestens eine Kohlenwasserstoffgruppe gepfropft ist, die mindestens eine Carboxyfunktion aufweist.

13. Zusammensetzung nach Anspruch 12, **dadurch gekennzeichnet, dass** das Silicon die folgende Einheit (IX) enthält: 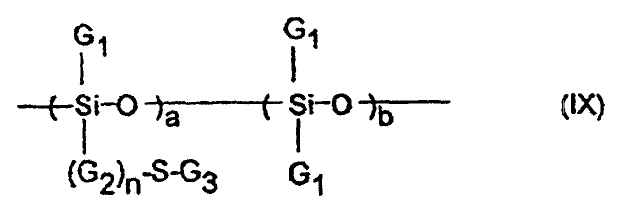 worin die Gruppen G₁ Wasserstoff, C₁₋₁₀-Alkyl oder Phenyl; die Gruppen G₂ C₁₋₁₀-Alkylen; G₃ eine anionische Polymergruppe, die bei der (Homo)polymerisation mindestens eines anionischen Monomers mit ethylenischer Doppelbindung gebildet wird; n 0 oder 1; a 0 oder eine ganze Zahl im Bereich von 1 bis 50; und b eine ganze Zahl im Bereich von 10 bis 350 bedeuten.

14. Zusammensetzung nach einem der Ansprüche 1 bis 10, **dadurch gekennzeichnet, dass** das Silicon mindestens eine Einheit der Formel (IV) aufweist:
ZRₐSiO_{(3-a)/2} (IV),
wobei die Gruppe Z eine Gruppe der folgenden Formel (V) ist: 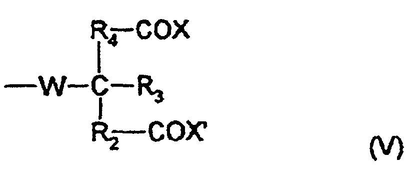 worin:
- die Gruppen W, R₂ und R₄, die identisch oder voneinander verschieden sind, unter einer kovalenten Bindung oder einer geradkettigen oder verzweigten C₁₋₆-Alkylengruppe ausgewählt sind, die gegebenenfalls eine Hydroxygruppe enthalten kann,
- die Gruppe R₃ Wasserstoff oder eine geradkettige oder verzweigte C₁₋₆-Alkylgruppe bedeutet,
- die Gruppen X und X', die identisch oder voneinander verschieden sind, unter den Gruppen OM, NR₅R₆ und OR₇ ausgewählt sind,
- M ein Wasserstoffatom, ein Alkalimetall (beispielsweise Na⁺, K⁺), NH₄⁺ oder eine Ammoniumgruppe bedeutet, die einen Rest umfasst, der von basischen Aminosäuren, wie Lysin, Arginin, Sarcosin, Ornithin oder Citrullin, oder Aminoalkoholen abgeleitet ist, wie Monoethanolamin, Diethanolamin, Triethanolamin, Glucamin, N-Methylglucamin oder 3-Amino-1,2-propandiol,
- die Gruppen R₅ und R₆, die identisch oder voneinander verschieden sind, unter Wasserstoff und den geradkettigen oder verzweigten C₁₋₆-Alkylgruppen ausgewählt sind, oder die Gruppen R₅ und R₆ gemeinsam einen 5- oder 6-gliedrigen Heterocyclus bilden, wie Morpholin,
- die Gruppe R₇ unter den geradkettigen oder verzweigten C₁₋₃₀-Alkylgruppen ausgewählt ist,
- wobei mindestens eine der Gruppen X oder X' OM bedeutet;
und wobei die Gruppen R der Formel (IV), die identisch oder voneinander verschieden sind, unter den C₁₋₁₀-Alkylgruppen, C₁₋₁₀-Fluoralkylgruppen und C₆₋₁₂-Arylgruppen ausgewählt sind und a 0, 1, oder 2 bedeutet.

15. Zusammensetzung nach einem der vorhergehenden Ansprüche, **dadurch gekennzeichnet, dass** die Zusammensetzung das Silicon oder Gemisch von Siliconen in einer relativen Gewichtsmenge von 0,01 bis 10 % und noch vorteilhafter von 0,05 bis 5 % enthält.

16. Zusammensetzung nach dem vorhergehenden Anspruch 15, **dadurch gekennzeichnet, dass** der relative Gewichtsanteil des Silicons oder Gemisches von Siliconen im Bereich von 0,05 bis 5 % liegt.

17. Zusammensetzung nach einem der vorhergehenden Ansprüche, **dadurch gekennzeichnet, dass** sie das Polykondensat, das mindestens eine Polyurethansequenz und/oder Polyharnstoffsequenz enthält, in einer relativen Gewichtsmenge von 1 bis 15 % und noch vorteilhafter von 2 bis 8 % enthält.

18. Zusammensetzung nach einem der vorhergehenden Ansprüche, **dadurch gekennzeichnet, dass** sie das Polykondensat, das mindestens eine Polyurethansequenz und/oder Polyharnstoffsequenz enthält, in einer relativen Gewichtsmenge von 2 bis 8 % enthält.

19. Zusammensetzung nach einem der vorhergehenden Ansprüche, **dadurch gekennzeichnet, dass** sie über einen Pumpflakon oder einen Aerosolbehälter abgegeben wird.

20. Zusammensetzung, die über eine Aerosolvorrichtung abgegeben wird, nach Anspruch 19, **dadurch gekennzeichnet, dass** sie (i) ein organisches Lösungsmittel in einer relativen Konzentration von 10 bis 50 Gew.-% enthält.

21. Zusammensetzung, die über eine Aerosolvorrichtung abgegeben wird, nach Anspruch 20, **dadurch gekennzeichnet, dass** sie (i) ein organisches Lösungsmittel in einer relativen Konzentration von 10 bis 25 Gew.-% enthält.

22. Zusammensetzung, die über eine Aerosolvorrichtung abgegeben wird, nach Anspruch 21, **dadurch gekennzeichnet, dass** sie (ii) ferner ein Treibgas in einer auf das Gewicht bezogenen relativen Konzentration von 15 bis 85 % enthält.

23. Zusammensetzung, die über eine Aerosolvorrichtung abgegeben wird, nach Anspruch 22, **dadurch gekennzeichnet, dass** sie (ii) ferner ein Treibgas in einer auf das Gewicht bezogenen relativen Konzentration von 25 bis 60 % enthält.

24. Zusammensetzung, die über eine Aerosolvorrichtung abgegeben wird, nach Anspruch 23, **dadurch gekennzeichnet, dass** sie (ii) ferner ein Treibgas in einer auf das Gewicht bezogenen relativen Konzentration von 30 bis 50 % enthält.

25. Zusammensetzung nach einem der vorhergehenden Ansprüche, **dadurch gekennzeichnet, dass** sie ferner herkömmliche kosmetische Zusatzstoffe enthält, die unter den Fettsubstanzen, Verdickungsmitteln, beruhigenden Stoffen, Schaumverhütungsmitteln, Hydratisierungsmitteln, Antiperspirantien, Alkalisierungsmitteln, Farbmitteln, Pigmenten, Parfums, Konservierungsmitteln, grenzflächenaktiven Stoffen, Polymeren auf Kohlenwasserstoffbasis, ergänzenden flüchtigen oder nicht flüchtigen Siliconen, Proteinen und Vitaminen ausgewählt sind.

26. Zusammensetzung nach einem der vorhergehenden Ansprüche, **dadurch gekennzeichnet, dass** die Zusammensetzung ferner ein zusätzliches fixierendes Polymer enthält, das unter den nichtionischen, kationischen, anionischen und amphoteren fixierenden Polymeren ausgewählt ist.

27. Aerosolvorrichtung, die aus einem Behälter, der eine Aerosolzusammensetzung nach Anspruch 1 enthält, sowie einer Einrichtung zur Verteilung der Zusammensetzung besteht.

28. Verfahren zur Formgebung oder Festigung der Frisur, **dadurch gekennzeichnet, dass** es die Verwendung einer Zusammensetzung nach einem der Ansprüche 1 bis 26 umfasst.

29. Verwendung einer Zusammensetzung nach einem der Ansprüche 1 bis 26 zur Herstellung von Produkten für die Haarbehandlung zur Festigung oder Formgebung der Frisur.

## Claims

1. Hair composition comprising, in a cosmetically acceptable medium, in relative proportions by weight relative to the total weight of the composition, from 0.1 to 20% of a polycondensate comprising at least one polyurethane and/or polyurea sequence, **characterized in that** it also comprises from 0.01 to 20% of at least one partially or totally neutralized silicone comprising at least one carboxylic function or one of its salts or a mixture thereof, the polycondensate being formed by an arrangement of blocks obtained from:
(1) at least one compound which contains two or more than two active hydrogen atoms per molecule;
(2) at least one diol or a mixture of diols containing acid radicals or their salts;
(3) at least one di- or polyisocyanate.

2. Composition according to Claim 1, **characterized in that** the compounds (1) are chosen from the group consisting of diols, diamines, polyesterols and polyetherols, or a mixture thereof.

3. Composition according to Claim 1, **characterized in that** the compound (2) is a 2,2-hydroxymethylcarboxylic acid.

4. Composition according to Claim 1, **characterized in that** the compound (3) is chosen from the group consisting of hexamethylene diisocyanate, isophorone diisocyanate, toluylene diisocyanate, diphenylmethane 4,4'-diisocyanate, dicyclohexylmethane 4,4'-diisocyanate, methylenebis(p-phenyl) diisocyanate, methylenebis(4-cyclohexyl isocyanate), isophorone diisocyanate, toluene diisocyanate, 1,5-naphthalene diisocyanate, 4,4'-diphenylmethane diisocyanate, 2,2'-dimethyl-4,4'-diphenylmethane diisocyanate, 1,3-phenylene diisocyanate, 1,4-phenylene diisocyanate, mixtures of 2,4- and 2,6-toluene diisocyanate, 2,2'-dichloro-4,4'-diisocyanatodiphenylmethane, 2,4-dibromo-1,5-diisocyanatonaphthalene, butane 1,4-diisocyanate, 1,6-hexane diisocyanate and 1,4-cyclohexane diisocyanate.

5. Composition according to Claim 1, **characterized in that** the polycondensate is formed from at least one additional compound having a silicone skeleton and chosen from the group consisting of polysiloxanes, polyalkylsiloxanes or polyarylsiloxanes.

6. Composition according to Claim 5, **characterized in that** the polycondensate is formed from at least one additional compound having a silicone skeleton and chosen from the group consisting of polyethylsiloxanes, polymethylsiloxanes and polyphenylsiloxanes, optionally containing hydrocarbon-based chains grafted onto the silicon atoms.

7. Composition according to Claim 1, **characterized in that** the polyurethane and/or polyurea sequences of the polycondensate have a repeating base unit corresponding to the general formula I' below:
-X'-B-X'-CO-NH-R-NH-CO- (I')
in which:
- X' represents O and/or NH,
- B is a divalent hydrocarbon-based radical, this radical being substituted or unsubstituted, and
- R is a divalent radical chosen from alkylene radicals of aromatic type, C₁ to C₂₀ aliphatic radicals or C₁ to C₂₀ cycloaliphatic radicals, these radicals being substituted or unsubstituted.

8. Composition according to Claim 7, **characterized in that** B is a C₁ to C₃₀ divalent hydrocarbon-based radical.

9. Composition according to Claim 7, **characterized in that** the radical R is chosen from the group consisting of hexamethylene, 4,4'-biphenylenemethane, 2,4- and/or 2,6-tolylene, 1,5-naphthylene, p-phenylene and methylene-4,4-bis-cyclohexyl radicals and the divalent radical derived from isophorone.

10. Composition according to Claim 1, **characterized in that** the polycondensate has a repeating base unit corresponding to formula (II'):
-X'-P-X'-CO-NH-R-NH-CO- (II')
in which:
- P is a polysiloxane segment,
- X' represents O and/or NH, and
- R is a divalent radical chosen from alkylene radicals of aromatic type, C₁ to C₂₀ aliphatic radicals and C₁ to C₂₀ cycloaliphatic radicals, these radicals being substituted or unsubstituted.

11. Composition according to any one of the preceding claims, **characterized in that** the silicone is an organopolysiloxane comprising at least one unit corresponding to formula I: 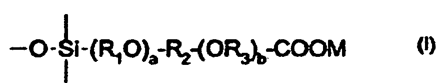 in which:
- R₁ and R₃ independently denote a linear or branched alkylene radical having from 2 to 20 carbon atoms;
- R₂ denotes a linear or branched alkylene radical having from 1 to 50 carbon atoms and optionally comprising a hydroxyl group;
- a represents 0 or 1;
- b is a number ranging from 0 to 200;
- M is chosen from the group comprising hydrogen, alkali metals or alkaline-earth metals, NH₄ and quaternary ammonium groups such as, in particular, mono-, di-, tri- or tetra(C₁-C₄ alkyl)ammonium groups.

12. Composition according to any one of Claims 1 to 10, **characterized in that** the silicone is formed by a main chain corresponding to the formula (≡Si-O-)ₙ onto which is grafted, inside the said chain as well as, optionally, on at least one of its ends, at least one hydrocarbon-based group comprising at least one carboxylic function.

13. Composition according to Claim 12, **characterized in that** the silicone comprises the unit IX below: 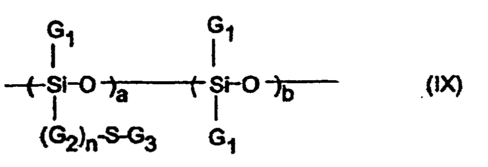 in which G₁ represents hydrogen or a C₁-C₁₀ alkyl radical or alternatively a phenyl radical; G₂ represents a C₁-C₁₀ alkylene group; G₃ represents an anionic polymer residue resulting from the (homo)polymerization of at least one anionic monomer containing ethylenic unsaturation; n is equal to 0 or 1; a is an integer which can be between 1 and 50; and b is an integer which can be between 10 and 350.

14. Composition according to any one of Claims 1 to 10, **characterized in that** the silicone comprises at least one unit of formula IV:
ZRₐSiO_{(3-a)/2} (IV)
in which Z is a radical corresponding to formula V below: 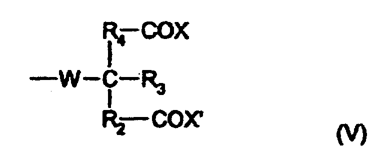 in which:
- W, R₂ and R₄, which may be identical or different, are chosen from a covalent bond and a linear or branched alkylene radical having from 1 to 6 carbon atoms which can comprise a hydroxyl group,
- R₃ denotes a hydrogen atom, a linear or branched C₁-C₆ alkyl radical,
- X and X', which may be identical or different, are chosen from the radicals OM, NR₅R₆ and OR₇,
- M denotes a hydrogen atom, an alkali metal (for example Na⁺, K⁺), NH₄⁺, the ammonium groups containing a residue chosen from the group consisting of basic amino acids such as lysine, arginine, sarcosine, ornithine or citrulline, and amino alcohols such as monoethanolamine, diethanolamine, triethanolamine, glucamine, N-methylglucamine and 3-amino-1,2-propanediol,
- R₅ and R₆, which may be identical or different, are chosen from the group consisting of hydrogen and linear or branched C₁-C₆ alkyls, or alternatively R₅ and R₆ can together form a 5- or 6-membered heterocycle such as morpholine,
- R₇ is chosen from linear or branched C₁-C₃₀ alkyl radicals,
- at least one of the groups X and X' denotes OM, the radicals R of formula IV, which may be identical or different, being chosen from the group consisting of C₁ to C₁₀ alkyl radicals, C₁ to C₁₀ fluoroalkyl radicals and C₆ to C₁₂ aryl radicals; a being an integer equal to 0, 1 or 2.

15. Composition according to any one of the preceding claims, **characterized in that** the relative proportion by weight of silicone or of silicone mixture is between 0.01 and 10% and more advantageously between 0.05 and 5%.

16. Composition according to the preceding Claim 15, **characterized in that** the relative proportion by weight of silicone or of silicone mixture is between 0.05 and 5%.

17. Composition according to any one of the preceding claims, **characterized in that** it comprises, in relative proportions by weight, between 1 and 15% of the polycondensate comprising at least one polyurethane and/or polyurea sequence, and more advantageously between 2 and 8%.

18. Composition according to any one of the preceding claims, **characterized in that** it comprises, in relative proportions by weight, between 2 and 8% of the polycondensate comprising at least one polyurethane and/or polyurea sequence.

19. Composition according to any one of the preceding claims, **characterized in that** it is conveyed via a pump-dispenser bottle or an aerosol device.

20. Composition according to Claim 19, which is conveyed via an aerosol device, **characterized in that** it comprises (i) an organic solvent in a relative concentration by weight of between 10 and 50%.

21. Composition according to Claim 20, which is conveyed via an aerosol device, **characterized in that** it comprises (i) an organic solvent in a relative concentration by weight of between 10 and 25%.

22. Composition according to Claim 21, which is conveyed via an aerosol device, **characterized in that** it also comprises (ii) a propellent gas in a relative concentration by weight of between 15 and 85%.

23. Composition according to Claim 22, which is conveyed via an aerosol device, **characterized in that** it also comprises (ii) a propellent gas in a relative concentration by weight of between 25 and 60%.

24. Composition according to Claim 23, which is conveyed via an aerosol device, **characterized in that** it also comprises (ii) a propellent gas in a relative concentration by weight of between 30 and 50%.

25. Composition according to any one of the preceding claims, **characterized in that** it also contains conventional cosmetic additives chosen from the group consisting of fatty substances, thickeners, softeners, anti-foaming agents, moisturizers, antiperspirants, basifying agents, dyes, pigments, fragrances, preserving agents, surfactants, hydrocarbon-based polymers, additional volatile or nonvolatile silicones, proteins and vitamins.

26. Composition according to any one of the preceding claims, **characterized in that** it contains at least one additional fixing polymer chosen from the group consisting of nonionic, cationic, anionic or amphoteric fixing polymers.

27. Aerosol device consisting of a container containing an aerosol composition according to Claim 1, as well as a means for distributing the said aerosol composition.

28. Process for shaping or maintaining the hairstyle, **characterized in that** it comprises the application of a composition in accordance with any one of Claims 1 to 26.

29. Use of a composition according to any one of Claims 1 to 26 for the manufacture of a hair composition, in order to maintain or shape the hairstyle.
